# EUROPEAN PATENT APPLICATION

(11) **EP 1 106 185 A1**
(43) Date of publication of application: **13.06.2001**
(21) Application number: 99204201.0
(22) Date of filing: 08.12.1999
(51) Int. Cl.: A61K 48/00

(54) **Method of administering adenovirus**

(71) Applicant: Introgene B.V., 2333 AL Leiden (NL)
(72) Inventor: Schraa, Edwin Oscar, 2311 ES Leiden (NL); Brus, Ronald Hendrik Peter, 2252 EB Voorschoten (NL); Esandi, Maria del Carmen, Bahia Blanca CP 8000 Buenos Aires (AR)
(74) Representative: Ottevangers, Sietse Ulbe

(57) **Abstract**

A method of reducing the risk associated with administering adenoviral particles to subjects undergoing adenoviral gene therapy. An extremely low titer of adenovirus neutralizing antibodies is believed to lead to the an acute virus-mediated cytotoxicity. Neutralizing antibodies prevent the hepatotoxic effects of adenoviruses administered by way of the hepatic artery. An improved adenoviral gene therapy method of the type involving the administration, at a certain point in time, of recombinant adenoviruses containing a gene of interest to a subject wherein the improvement includes, before administering the recombinant adenovirus containing the gene of interest, providing the subject with neutralizing antibodies directed to the recombinant adenovirus. In one aspect of the method, the subject is provided with the antibodies by administering to the subject adenovirus of the same type as the recombinant adenovirus to be administered, at a before the administration of the recombinant adenovirus, to induce in the subject an immune response against the adenovirus. Alternatively, when insufficient time exists, or the subject is immunocompromised, the subject is directly provided with the neutralizing antibodies by administering, to the subject, antibodies directed against the same serotype of adenovirus as the recombinant adenovirus or antibodies cross-reactive with the serotype of the recombinant adenovirus. In such a case, the antibodies are administered about one hour before the administration of the recombinant adenovirus containing the gene of interest. In this aspect of the invention, human antibodies or humanized antibodies are particularly preferred to be administered parenterally to the subject.

## Description

Technical Field: The invention relates to the field of recombinant DNA technology, more in particular to the field of gene therapy. In particular the invention relates to adenoviral gene therapy wherein a subject to undergo adenoviral gene therapy, the subject not having pre-existing antibodies to adenovirus is provided therewith.

Background: Gene therapy is a recently developed concept for which a wide range of applications can be and have been envisioned. In gene therapy a molecule carrying genetic information is introduced into some or all cells of a host, as a result of which the genetic information is added to the host in a functional format.

The genetic information added may be a gene or a derivative of a gene, such as a cDNA, which encodes a protein. This is a functional format in that the protein can be expressed by the machinery of the host cell.

The genetic information can also be a sequence of nucleotides complementary to a sequence of nucleotides (either DNA or RNA) present in the host cell. This is a functional format in that the added DNA (nucleic acid) molecule or copies made thereof *in situ* are capable of base pairing with the complementary sequence present in the host cell.

Applications include the treatment of genetic disorders by supplementing a protein or other substance which, because of the genetic disorder, is either absent or present in insufficient amounts in the host, the treatment of tumors and the treatment of other acquired diseases such as (auto)immune diseases, infections, etc.

As may be inferred from the foregoing, basically three different approaches exist in gene therapy: the first directed towards compensating for a deficiency in a (mammalian) host, the second directed towards the removal or elimination of unwanted substances (organisms or cells) and the third towards application of a recombinant vaccine (against tumors or foreign micro-organisms).

For the purpose of gene therapy, adenoviruses carrying deletions have been proposed as suitable vehicles for genetic information. Adenoviruses are non-enveloped DNA viruses. Gene-transfer vectors derived from adenoviruses (so-called "adenoviral vectors") have a number of features that make them particularly useful for gene transfer for such purposes. For example, the biology of the adenoviruses is characterized in detail, the adenovirus is not associated with severe human pathology, the virus is extremely efficient in introducing its DNA into the host cell, the virus can infect a wide variety of cells and has a broad host-range, the virus can be produced in large quantities with relative ease, and the virus can be rendered replication defective by deletions in the early-region 1 ("E1") of the viral genome.

The adenovirus genome is a linear double-stranded DNA molecule of approximately 36000 base pairs with the 55-kDa terminal protein covalently bound to the 5' terminus of each strand. The adenoviral ("Ad") DNA contains identical Inverted Terminal Repeats ("ITR") of about 100 base pairs with the exact length depending on the serotype. The viral origins of replication are located within the ITRs exactly at the genome ends. DNA synthesis occurs in two stages. First, the replication proceeds by strand displacement, generating a daughter duplex molecule and a parental displaced strand. The displaced strand is single stranded and can form a so-called "panhandle" intermediate, which allows replication initiation and generation of a daughter duplex molecule. Alternatively, replication may proceed from both ends of the genome simultaneously, obviating the requirement to form the panhandle structure.

During the productive infection cycle, the viral genes are expressed in two phases: the early phase, which is the period up to viral DNA replication, and the late phase, which coincides with the initiation of viral DNA replication. During the early phase only the early gene products, encoded by regions E1, E2, E3 and E4, are expressed, which carry out a number of functions that prepare the cell for synthesis of viral structural proteins (Berk, 1986). During the late phase the late viral gene products are expressed in addition to the early gene products and host cell DNA and protein synthesis are shut off. Consequently, the cell becomes dedicated to the production of viral DNA and of viral structural proteins (Tooze, 1981).

The E1 region of adenovirus is the first region of adenovirus expressed after infection of the target cell. This region consists of two transcriptional units, the E1A and E1B genes, which both are required for oncogenic transformation of primary (embryonic) rodent cultures. The main functions of the E1A gene products are 1) to induce quiescent cells to enter the cell cycle and resume cellular DNA synthesis, and 2) to transcriptionally activate the E1B gene and the other early regions (E2, E3, E4). Transfection of primary cells with the E1A gene alone can induce unlimited proliferation (immortalization), but does not result in complete transformation. However, expression of E1A in most cases results in induction of programmed cell death (apoptosis), and only occasionally immortalization (Jochemsen *et al.,* 1987). Co-expression of the E1B gene is required to prevent induction of apoptosis and for complete morphological transformation to occur. In established immortal cell lines, high level expression of E1A can cause complete transformation in the absence of E1B (Roberts *et al.,* 1985).

The E1B encoded proteins assist E1A in redirecting the cellular functions to allow viral replication. The E1B 55 kD and E4 33kD proteins, which form a complex that is essentially localized in the nucleus, function in inhibiting the synthesis of host proteins and in facilitating the expression of viral genes. Their main influence is to establish selective transport of viral mRNAs from the nucleus to the cytoplasm, concomittantly with the onset of the late phase of infection. The E1B 21 kD protein is important for correct temporal control of the productive infection cycle, thereby preventing premature death of the host cell before the virus life cycle has been completed. Mutant viruses incapable of expressing the E1B 21 kD gene-product exhibit a shortened infection cycle that is accompanied by excessive degradation of host cell chromosomal DNA (deg-phenotype) and in an enhanced cytopathic effect (cyt-phenotype) (Telling *et al*., 1994). The deg and cyt phenotypes are suppressed when in addition the E1A gene is mutated, indicating that these phenotypes are a function of E1A (White *et al.,* 1988). Furthermore, the E1B 21 kDa protein slows down the rate by which E1A switches on the other viral genes. It is not yet known through which mechanisms E1B 21 kD quenches these E1A dependent functions.

Vectors derived from human adenoviruses, in which at least the E1 region has been deleted and replaced by a gene of interest, have been used extensively for gene therapy experiments in the pre-clinical and clinical phase. Generally, in the pre-clinical trials using mouse and baboon models, the dosage range used has been between two billion and 6 X 10¹¹ viral particles per kilogram body mass, administered intrahepatically. However, there was a dose related toxicity exhibited.

There are a number of features of adenoviruses that make them particularly useful for the development of gene-transfer vectors for human gene therapy:
1. The adenovirus genome is well characterized. It consists of a linear double-stranded DNA molecule of approximately 36000 base pairs. The adenovirus DNA contains identical Inverted Terminal Repeats (ITR) of approximately 90-140 base pairs with the exact length depending on the serotype. The viral origins of replication are within the ITRs exactly at the genome ends;
2. The biology of the adenoviruses is characterized in detail; the adenovirus is not associated with severe human pathology in immunocopetent individuals
3. The virus is extremely efficient in introducing its DNA into the host cell; the virus can infect a wide variety of cells and has a broad host-range;
4. The virus can be produced at high virus titers in large quantities;
5. The virus can be rendered replication defective by deletion of the early-region 1 (E1) of the viral genome (Brody et al, 1994). Most adenoviral vectors currently used in gene therapy have a deletion in the E1 region, where novel genetic information can be introduced.

Based on these features, preferred methods for *in vivo* gene transfer into human target cells, make use of adenoviral vectors as gene delivery vehicles. Adenoviral *in vivo* gene transfer efficiency was shown in preclinical studies (Eastham, J.A., *et al.,* 1996; Tang, D.C. et al., 1994; Esandi, M.C., *et al.,* 1997; Vincent, A.J., *et al.* 1996 a and b).
However, there are still problems associated with the therapeutic use of adenoviral vectors in humans. One problem is the occurrence of virus mediated cellular toxicity. Several studies in animal models indicate that gene transfer using adenovirus vectors *in vivo* may be limited by the development of anti-adenovirus immunity (Yang, Y. and et al, 1994; Mack, C.A., *et al 1997;* Bouvet, M., *et al.,* 1998; Kaplan, J.M. and A.E. Smith, 1997; Schulick, A.H., *et al.,* 1997).
A recent clinical trial on dose escalation studies with local administration of recombinant adenovirus to the liver showed the development of grade III or higher hepatocellular toxicity (Batshaw et al. 1999).
According to basic pharmacology, the toxicity of a treatment largely depends on the dose given. Already in 1995, Knowles et al. demonstrated a significant toxicity at doses necessary to achieve high levels of transgene expression (Knowles et al. 1995). In small animal models, several studies report toxicity seen after virus administration. A dose of 4E9 pfu in mice caused dramatic elevation of liver-specific enzyme levels, which was dose-related (Connelly et al. 1996). Morral et al. reported that a rapid decrease in expression with high dose of vector was most consistent with a non-immune-mediated toxic effect (Morral et al. 1997). Substantial elevations of hepatocyte enzymes in serum were demonstrated at the two highest doses, being 5.4E9 and 1.6E10 pfu (Morral et al. 1997). E1- and E2a-deleted vectors both led to dose-dependent transient thrombocytopenia, elevations in liver enzymes in the serum, and hepatocyte proliferation followed by immune cell infiltration and hepatocyte hypertrophy (O'Neal et al. 1998). It was suggested that hepatocyte toxicity was related primarily to viral entry and expression, rather than to the presence of non-infectious particles (O'Neal et al. 1998). In rabbits intravenous administration of recombinant adenoviruses caused thrombocytopenia and erythroblastosis to arise within 24 hours (Cichon et al. 1999).
Early liver toxicity as determined by serum glutamic pyruvic transaminase elevation and inflammatory cell infiltrates appeared to be dependent on adenovirus-mediated hepatocyte apoptosis (Lieber et al. 1997). Schowalter et al. claimed that this apoptotic process might be caused by an antigen-dependent immunity due to persistent expression of the transgene (Schowalter et al. 1999). Muruve et al. reported that high titres of adenoviral vectors caused significant hepatic necrosis and apoptosis following systemic administration (Muruve et al. 1999). Reduced alanine_/aspartate-aminotransferase levels and attenuated adenovirus-induced hepatic injury after neutrophil depletion and MIP-2 antagonism suggested that early injury was largely due to chemokine production and neutrophil recruitment (Muruve et al. 1999). Nevertheless, Gagandeep et al. demonstrated virus-mediated apoptosis in SCID mice lacking T, B, NK and LAK cells (Gagandeep et al. 1999).
In addition in non-human primates, virus administration in the portal vein resulted in histopathological changes in the liver, like portal and lobular inflammation, apoptosis and mitosis (Nunes et al. 1999). A rough dose-response could be seen (Nunes et al. 1999). With increasing vp dose, as was the design of the above-mentioned trial, more adverse effects would have been encountered.
Exposure to wild-type adenoviruses is very common in humans. The vast majority of individuals have had previous exposure to adenoviruses, especially the well investigated adenovirus serotypes 5 and type 2 (Ad5 and Ad2). This exposure has resulted in immune responses against many types of adenoviruses. The prior art teaches that this phenomenon of pre-existing antibodies may interfere with in vivo gene transfer using recombinant adenoviral vectors (Yang et al 1994 and 1995; Dai et al 1995; Gilgenkrantz 1995). Humoral responses to adenovirus result in neutralizing antibodies. The art mentioned above shows that protective immunity in humans, due to exposure of the human population to adenovirus, is seen as an obstacle to adenovirus-mediated gene therapy.
There are other prior art data in the literature that suggest that the presence of neutralizing antibodies does not prevent tumor gene transfer. Li *et al* have reported that multiple administration of adenovirus vectors intratumorally are feasible and efficacious in animals with pre-existing Nab against adenovirus. Others even suggest that in humans the systemic anti-adenovirus Nab response differs depending on the target organ and is independent of the viral vector dose (Molnar-Kimber, K.L., *et al.,* 1998; Harvey, B.G., *et al.,* 1999).
Bramson et *al* have reported that neutralizing antibodies against adenovirus do not prevent tumor regression after intratumoral delivery of a vector expressing IL-12 but inhibits virus dissemination. This study suggests that Ad immunity is not a barrier for cancer gene therapy. In non-immunized animals the virus was found to disseminate to the liver, while a 1000-fold reduction of this dissemination was observed in immune mice. These authors hypothesize that poorly vascularized solid tumors are detached from the systemic circulation and then have less contact with neutralizing antibodies than other organs such as liver. Tragedy has struck in one recent human study evaluating patients with partial OTCD (ornithine transcarbamylase disease). In that study, patients were to be started with 2 X 10⁹ adenoviral particles per kilogram body mass, with subsequent one-half log increases. A relatively healthy OTCD patient was intrahepatically administered (by hepatic intraarterial catheter) approximately 3 X 10¹³ adenoviral particles. He soon died.

### Summary of the Invention

The invention includes a method of reducing the risk associated with administering adenoviral particles to subjects undergoing gene therapy. An extremely low titer of adenovirus neutralizing antibodies is believed to lead to the an acute virus-mediated cytotoxicity. Neutralizing antibodies prevent the hepatotoxic effects of adenoviruses administered by way of, for example, the hepatic artery.
The invention thus includes an improvement in an adenoviral gene therapy method of the type involving the administration, at a certain point in time, of recombinant adenoviruses containing a gene of interest to a subject believed to be in need thereof, wherein the improvement includes, before administration of the recombinant adenovirus containing the gene of interest, providing the subject with antibodies directed to an adenovirus of the same serotype or cross-reactive with the same serotype as the recombinant adenovirus containing the gene of interest.
In one aspect of the method, the subject is provided with the antibodies by administering, to the subject, adenovirus of the same type as the recombinant adenovirus to be administered, at a before the administration of the recombinant adenovirus, to induce in the subject an immune response against the adenovirus.
Alternatively, when insufficient time exists, or the subject is immunocompromised, the subject is directly provided with the neutralizing antibodies by administering, to the subject, antibodies directed against the same serotype of adenovirus as the recombinant adenovirus or antibodies cross-reactive with the serotype of the recombinant adenovirus. In such a case, the antibodies are administered about one hour before the administration of the recombinant adenovirus containing the gene of interest. In this aspect of the invention, human antibodies or humanized antibodies are particularly preferred to be administered parenterally to the subject. The invention also includes a kit of parts for gene delivery to a recipient cell in a host, using a gene delivery vehicle to which a humoral response can be raised, comprising a nucleic acid comprising a gene to be delivered to said recipient cell and further comprising a second composition of a second gene delivery vehicle essentially identical to said first gene delivery vehicle, but preferably lacking said gene to be delivered. In this kit, the second composition is a vaccine and the first composition is a composition for gene therapy. The vaccine is to be administered at a time before said gene therapy composition at a moment in time sufficient for the host to raise a neutralising humoral response to said second gene delivery vehicle. The second gene delivery may comprise a nucleic acid encoding an indifferent protein. The gene delivery vehicle may be of adenoviral origin.
The invention also a method for delivering a gene of interest to a recipient cell in a host using a gene delivery vehicle comprising a nucleic acid comprising said gene of interest, said method comprising administering to a host a vaccine composition comprising a gene delivery vehicle lacking said gene to be delivered, allowing for a neutralizing humoral response to be raised by said host to said gene delivery vehicle lacking said gene to be delivered and administering a composition for gene therapy comprising essentially the same gene delivery vehicle having a nucleic acid comprising said gene to be delivered in an amount greater than the amount which can be neutralised by said humoral response.

Further the invention includes a method for avoiding or diminishing liver toxicity in a host of a gene delivery composition upon administration, comprising administering to a host a vaccine composition comprising a gene delivery vehicle, preferably lacking said gene to be delivered, allowing for a neutralizing humoral response to be raised by said host to said gene delivery vehicle lacking preferably said gene to be delivered and administering a composition for gene therapy comprising essentially the same gene delivery vehicle having a nucleic acid comprising said gene to be delivered in an amount greater than the amount which can be neutralized by said humoral response.

The invention includes a method for the preparation of the aforementioned kit of parts, comprising preparing at least one gene delivery vehicle by inserting a gene of interest in a nucleic acid to be delivered to a host cell, packaging said nucleic acid in a vehicle capable of entering a host cell and bringing a resulting gene delivery vehicle in a medium suitable for administration to a host.

### Brief Description of the Figures

The following figures and drawings may help to understand the invention:

FIG. 1- Evaluation of luciferase activity after it injection of IG.Ad.AdApt.luc. Left L42 tumors were injected with IG.Ad.AdApt.luc at different doses: 10⁹, 10⁸ and 10⁷ IU/100µl. After 48 hours, luciferase activity was measured in the lysates organs (n=3). Vertical bars indicate SD. results are expressed in RLU (luciferase activity units) per mg of protein. Luciferase determination protocol was according to Fortunati et al..

FIG. 2- Growth curves of injected L42 tumors following treatment with IG.Ad.CMV.rIL-3. Arrows indicate daily vector administration (2.5x10⁸ IU) into left tumor from day 11 till 21.

FIG. 3- Growth curves of contralateral L42 tumors following treatment with IG.Ad.CMV.rIL-3. Arrows indicate daily vector administration (2.5x10⁸ IU) into left tumor from day 11 till 21.

FIG. 4- Leukocyte levels in naive and immune rats after IG.Ad.CMV.rIL-3 treatment.

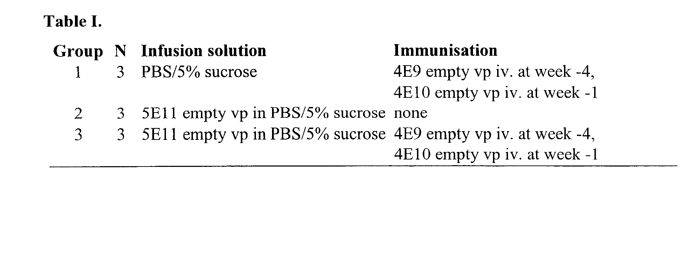

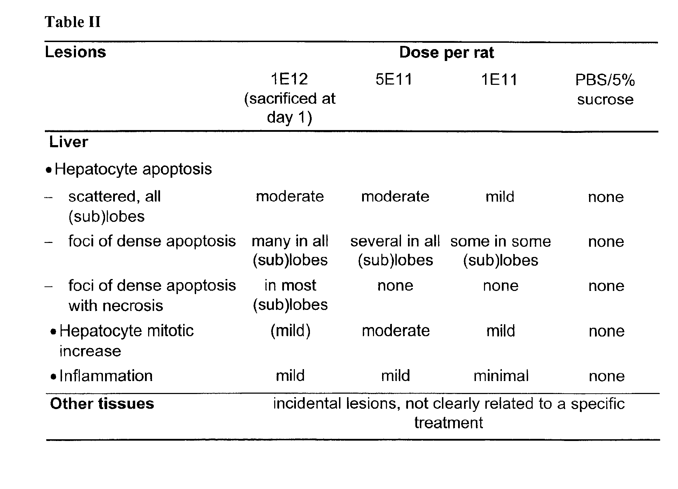

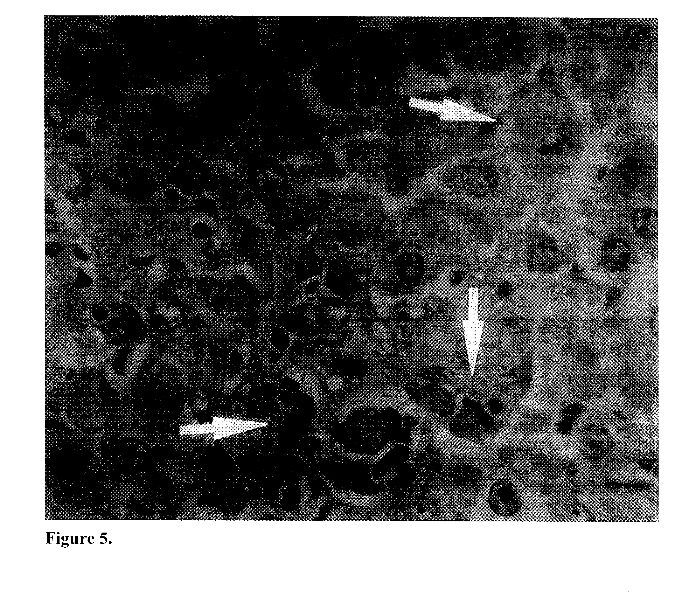

FIG. 5 depicts apoptotic and mitotic hepatocytes.

FIG. 6 depicts ALAT levels before and 3 days after HAI in all groups.

FIG. 7 depicts ASAT levels before and 3 days after HAI in all groups.

FIG. 8 depicts yGT levels before and 3 days after HAI in all groups.

FIG. 9 depicts AP levels before and 3 days after HAI in all groups.

FIG. 10 depicts TBIL levels before and 3 days after HAI in all groups.

FIG. 11 depicts ALB levels before and 3 days after HAI in all groups.

### Detailed Description of the Invention

A preferred method of adenoviral gene therapy involves the administration of recombinant adenovirus containing a gene of interest to a subject. The method includes first determining the serotype of recombinant adenovirus to be administered to the subject; administering to the subject, before administration of the recombinant adenovirus containing the gene of interest, at least one dose of adenovirus, the at least one dose of adenovirus being administered in an amount sufficient to elicit an immune response to the adenovirus in the subject. The at least one dose of adenovirus is preferably selected from the group of adenoviruses consisting of adenoviruses of the same serotype as the recombinant adenovirus and adenoviruses of a group which produces antibodies cross-reactive with the recombinant adenovirus; allowing the subject to mount an immune response to the first dose of adenovirus; and administering the recombinant adenovirus containing the gene of interest to the subject in an amount sufficient to allow the gene of interest to be taken up into the subject's cells.

For the pre-vaccination step, "empty vectors" of adenovirus are preferably used. At the least, adenovirus deleted for E1 should be used, so as to render the pre-vaccination adenovirus replication incompetent. Preferably, deletions of the E2, E3, and E4 region also take place.

The pre-vaccination step adenovirus is administered in a dosage sufficient to elicit an immune response, but insufficient to cause hepatotoxicity. Illustratively, dosages of 1 X 10⁹ to 1 X 10¹¹ viral particles per kilogram body mass may be administered.

In order to ensure that the subject will have sufficient anti-adenoviral antibodies to withstand the gene therapy with recombinant adenovirus, the pre-vaccination dose of adenovirus will be administered at least fourteen days before the gene therapy with the recombinant adenovirus is undertaken. A second "booster" dose may be administered after another week to a week and a half after the first.

A second preferred method of adenoviral gene therapy includes a) determining the serotype of recombinant adenovirus to be administered to the subject; b) administering to the subject, approximately one hour before the administration of the recombinant adenovirus containing the gene of interest, antibodies selected from the group consisting of antibodies directed against adenoviruses of the same serotype as the recombinant adenovirus and antibodiescross-reactive with the recombinant adenovirus; and c) administering the recombinant adenovirus containing the gene of interest to the subject.

In this embodiment, sufficient human or humanized anti-adenoviral antibodies are administered to the subject to prevent the toxic effects of the gene therapy, but insufficient to prevent the gene therapy from being efficacious.

As previously stated, all adenovirus vectors currently used in gene therapy are believed to have a deletion in the El region, where novel genetic information can be introduced. The E1 deletion is intended to render the recombinant virus replication defective (Stratford-Perricaudet and Perricaudet, 1991). It has been demonstrated that recombinant adenoviruses are able to efficiently transfer recombinant genes to the rat liver and airway epithelium of rhesus monkeys (Bout *et al.,* 1994b; Bout *et al.,* 1994a). In addition, it has been observed a very efficient *in vivo* adenovirus mediated gene transfer to a variety of tumor cells *in vitro* and to solid tumors in animals models (lung tumors, glioma) and human xenografts in immunodeficient mice (lung) *in vivo* (reviewed by Blaese *et al.,* 1995).

In the inventive method, the nucleic acid may be based on or derived from an adenovirus. Furthermore, the vehicle may comprise adenoviral structural proteins. In one preferred embodiment, at least one vehicle comprises proteins from adenoviruses of different serotypes. Furthermore, the nucleic acid may comprise at least one ITR and a packaging signal based on or derived from an adenovirus. Furthermore, the nucleic acid need not encode functional structural adenoviral proteins. The nucleic acid encodes E2A adenoviral gene product. Such an E2A gene product may be temperature sensitive. The packaging may occur in a packaging cell. The packaging cell may be derived from or based on a primary cell, such as PER.C6 (commercially available from IntroGene, bv of Leiden, NL or see published PCT International patent application WO 97/00326). The nucleic acid may comprise the gene of interest is produced by a recombination step from two adenoviral vectors.
The adenovirus is preferably adenovirus serotype 5 or serotype 35.

The invention may also be useful in gene therapy using other viruses such as adeno-associated virus, retrovirus, and other viruses useful in gene therapy.

The invention is further explained with the use of the following illustrative Examples.

### EXAMPLES

### Vectors

The vector used in the liver infusion experiment was a production produced adeno E1 virus, Ig.Ad5.CLIP.empty. The virus characteristics were: Batch B269-032, 8.53E11 vp/ml, 2E10 IU/ml.

### Description of the experimental design

For animal experiments, rats were used. Male inbred F344 rats, weighing 30 and 350 grams were used. Rats are obtained from Harlan-CPB (Austerlitz, the Netherlands). The rats were fed with a standard laboratory chow and water and were kept under standard laboratory conditions.

A hepatic artery infusion model ("HAI") was developed. Essential to this experiment is the slow pace of infusion to allow mixture of blood and virus before entering the liver. In a pilot study, 1E11, 5E11 and 1E12 empty viral particles ("vp") were infused (HAI model) in a naive rat to determine a virus dose that induced toxicity. After 3 days, the liver, lungs and kidney were removed and analyzed for apoptosis, necrosis, inflammation, thrombi or endothelial damage. When the dose was determined, the HAI was performed in immunized rats. In this experiment, plasma and serum was taken to measure the neutralising antibody titre and liver damage parameters, like alanine amino transferase (ALAT), aspartate amino transferase (ASAT), etc., and blood cell counts. After 3 days, the liver with its arterial vasculature, lungs, kidney, heart, mediastinal lymph nodes, spleen, pancreas, stomach and duodenum was removed and analysed for apoptosis, necrosis, acute inflammation, endothelial cell damage, leukocyte marginalisation, and thrombi.

### Hepatic arterial infusion model

The rat was anaesthetised with ether and the abdomen opened by longitudinal incision. To mimic the human protocol as much as possible, the rat hepatic arterial infusion model was developed. The gastroduodenal artery of the rat was isolated and ligated as close to the pancreas as possible. A clamp was placed on the common hepatic artery below the portal vein and a traction suture on the gastroduodenal artery near the small hepatic artery. The gastroduodenal artery was canulated retrograde with Teflon tubing (0.2x0.4mm). The clamp and traction suture were removed, after which 590 _l of PBS/5% sucrose containing the adenoviral vector was infused into the hepatic artery during 2 minutes. This flow rate allowed arterial blood flow to the liver. After infusion, the canule was removed and the gastroduodenal artery ligated.

### Sensitisation

Rats were sensitized against Ad 5 with 4E9 empty virus particles by an intravenous injection 4 weeks before HAI. One week prior to HAI a boost (4E10 vp) were given to ensure high antibody titres.

### Neutralising antibodies:

Rat plasma diluted serially and mixed with the luciferase recombinant virus (± MOI 100) was loaded on 96-wells plate coated with A549 cells. After 48 hours, luciferin was added and luminescence measured by the SteadyGlow assay. The neutralising antibody titre (NAb) was defined as the titre that resulted in a luminescence reduction of 50% of the positive control.

### Liver damage parameters

In rat plasma, ALAT, ASAT, alkaline phosphatase (AP), gamma-glutamyl transferase (_GT), total bilirubin (TBIL) and albumin (ALB) levels were measured as an indication of hepatocellular injury and liver function.

At necropsy, gross lesions if present were described and sampled. Routinely, the following tissues were sampled:

The liver, the common hepatic artery and point of infusion were taken out en bloc. Small incisions were made in all lobes. A small piece of the median lobe was snapfrozen for eventual further studies. The whole liver was fixated in ample 10% buffered formalin.

The lungs were insufflated with 10% buffered formalin and taken out as a block with heart, thymus and mediastinum for further fixation in ample formalin. In addition, the spleen/pancreas/stomach/duodenum block and right kidney were removed for fixation in formalin.

After fixation, all tissues were further trimmed and processed to routinely stained HPS sections at the histology lab. The liver sections nearest to the entry of the hepatic artery were numbered. All sections were examined microscopically by the pathologist for signs of toxicity. The expected signs of toxicity were apoptosis, necrosis, acute inflammation, endothelial damage and thrombi for liver sections, leukocyte marginalisation and thrombi for lung sections, focal necrosis for mediastinal lymph nodes sections, a rise in (endocardial) leukocytes for heart right ventricle and spleen sections, thrombi for kidney sections and procedure related ischaemic damage in stomach, duodenum and pancreas.

In a pilot study, 1E11, 5E11 and 1E12 empty vp were infused (HAI model) in a naive rat to determine a virus dose that induces toxicity. PBS/5% sucrose was used as a control. After 3 days, the liver, lungs and kidney were removed and analysed for signs of toxicity.

When the dose that induced hepatocellular toxicity was determined, 3 groups of 3 rats each undergo HAI. The infusion and immunisation of the rats in the separate groups were performed as shown in Table I. Anti-Ad5 antibody levels were determined before HAI to select animals with high titres (≥ 1024) for groups 1 and 3.

At day 0, the HAI was performed. The animals were sacrificed at day 3 for necropsy. Just before and at necropsy, blood is collected for leukocyte, thrombocyte and platelet counts and enzyme measurements.

### Biodistribution of an adenoviral vector following intratumoral administration in naive and immune rats

An adenoviral vector carrying the luciferase reporter gene (IG.Ad.AdApt.Luc) was used to determine the pattern of vector distribution and transgene expression following intratumoral administration (it). The tumor employed is a non immunogenic transplantable rat bronquial carcinoma, L42.

Naive and immune rats (Wag/Rij strain, 6-8 weeks old) bearing subcutaneous L42 tumors were used in this experiment. To evaluate virus dissemination to distant tumors, animals had bilateral subcutaneous tumors.

On day 0, tumor pieces were implanted subcutaneous in Wag/Rij rats (n=18) according to Esandi *et al.* [15]To induce the production of neutralizing antibodies against Ad5, rats (n=9) received an intravenous injection of 10⁸ IU of IG.Ad5.CLIP.empty virus on day 0. A second immunization was performed on day 13 using the same dose, virus vector and route of administration. On day 18, different doses (10⁹, 10⁸ and 10⁷ IU) of the luciferase vector was administered it. according to the following scheme:
Group 1 (n=3): immune animals bearing L42 tumors injected it. (left tumor) with IG.Ad.Adapt.Luc (10⁹IU/100µl)
Group 2 (n=3): immune animals bearing L42 tumors injected it. (left tumor) with IG.Ad.Adapt.Luc (10⁸IU/100µl)
Group 3 (n=3): immune animals bearing L42 tumors injected it. (left tumor) with IG.Ad.Adapt.Luc (10⁷IU/100µl)
Group 4 (n=3): naive animals bearing L42 tumors injected it. (left tumor) with IG.Ad.Adapt.Luc (10⁹IU/100µl)
Group 5 (n=3): naive animals bearing L42 tumors injected it. (left tumor) with IG.Ad.Adapt.Luc (10⁸IU/100µl)
Group 6 (n=3): naive animals bearing L42 tumors injected it. (left tumor) with IG.Ad.Adapt.Luc (10⁷IU/100µl)
The characteristics of the virus batch were: IG.Ad.AdApt.Luc (B358-044), titer: 2.6x10¹⁰ IU/ml, VP/IU ratio: 12

The same day of Luciferase vector administration, blood samples were collected to measure antibody titers. Two days later animals were sacrificed and organs were harvested for determination of luciferase activity. The antibody titers at the moment of Ad.luc injection were more than 2000 for all the animals (range: 2000-8000).

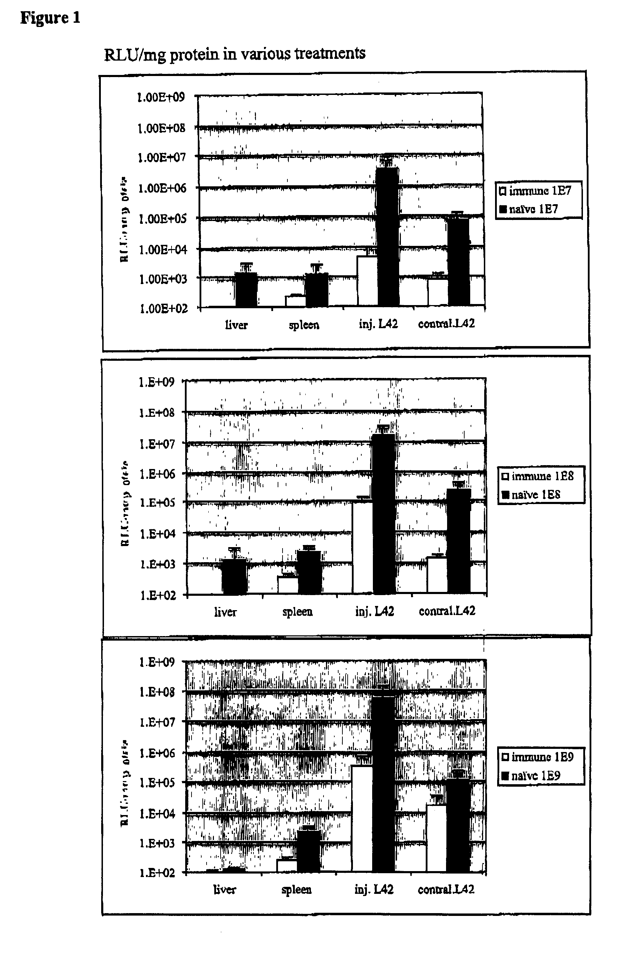

We have observed gene transfer inhibition to the liver and spleen of the immune rats. High titers of neutralizing antibodies did not prevent luciferase expression in the tumor (Fig.1). Surprisingly, after intratumoral injection, luciferase activity was found in the contralateral tumors in naive and immune rats (fig 1). This might indicate that gene transfer to distant tumors is not prevented by Nab.

### Effect of neutralizing antibodies on Ad5 mediated cancer gene therapy

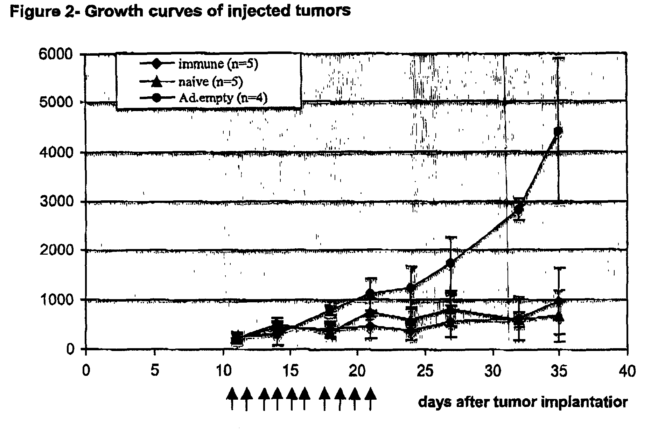

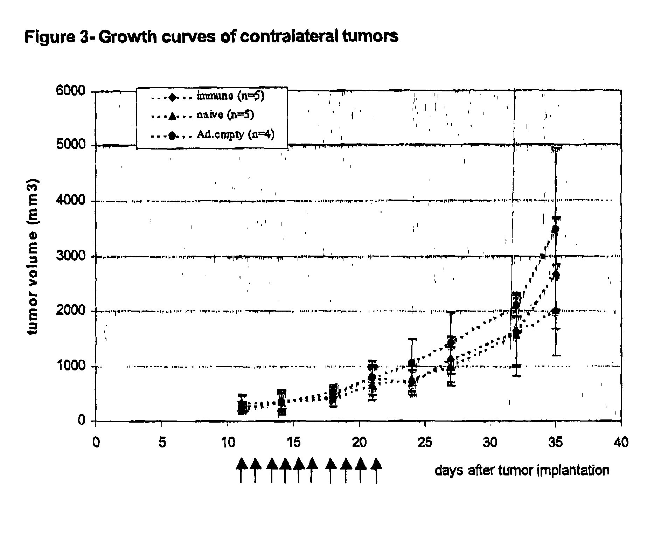

We previously reported regression of an established rat bronchial carcinoma after intratumoral injections of rIL-3b "cracked" adenoproducer cells (Esandi et al. 1998). Besides the antitumor effect, we also have found side effects associated to the IL-3 treatment represented by leukocytosis, extramedullary hematopoiesis reflected by enlarged spleen and liver (Fortunati et al, 1996). To investigate the effect of neutralizing antibodies on the outcome of Ad5-based immunotherapy, naive and immune rats bearing subcutaneous bilateral L42 tumors received Ad.IL-3 treatment. Rats were immunized by iv (n=4) and sc (n=2) injection of 10⁸ IU of Ad.CLIP.empty 11 days before vector injection. Prior to the IL-3 treatment, Ad5 antibody titers were >1000 for the iv immunized rats and 200 for the sc immunized rats. Naive and immune rats received 2.5x10⁸ IU of IG.Ad.CMV.rIL-3/day during 10 consecutive days. Tumor sizes were monitored twice a week. As control group, naive rats (n=4) received the same virus particle dose of IG.Ad.CLIP.empty. Growth delay of the injected tumors was observed in the naive and immune groups comparing to the control group (Fig.2). Non contralateral effect was detected after treatment (Fig 3).

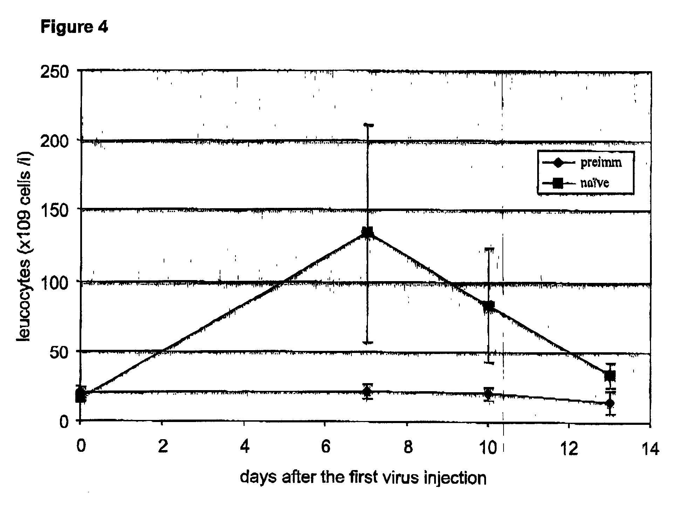

Side effects were monitored during the IL-3 treatment in the naive and immune rats. Leukocytosis was only observed in the naive group. Normal white blood cells counts were detected in the blood samples of the immune rats (Fig. 4). One rat from the naive and another of the immune group were sacrificed after the tenth injection to look for gross pathology. Hepato and splenomegaly were observed in the naive rat. No abnormalities were found in the immune rat.

### EXAMPLE II

### Toxicity pilot

The animal that received 1E12 vp in the hepatic artery showed serious signs illness. It was therefore sacrificed at day 1 after HAI. Further a dose-response physical appearance could be seen. After necropsy, the liver was fixated and all seven lobes were examined histologically. The pathological scoring is depicted in Table II.

The virus dose of 5E11 vp was chosen for the experiment, because clear signs of toxicity were found pathologically. Therefore, a decrease of toxicity, as hypothesised, would be detected easily. Another advantage was that if immunisation increased toxicity this could also be seen.
The dose-response toxicity seen in liver lesions allowed to develop a pathological grading. The scoring was simplified to lesions scored as none (-) to severe (+++). The different stages of pathological abnormalities are shown in Table III.

### Toxicity experiment

No unexpected deaths were encountered for the whole course of the experiment, meaning immunisation, HAI and the days following HAI. No differences were found in physical appearance of the rats treated with either PBS/5% sucrose or 5E11 empty virus particles in both groups. No significant alterations were found in leukocyte, red blood cell and platelets counts between all groups.

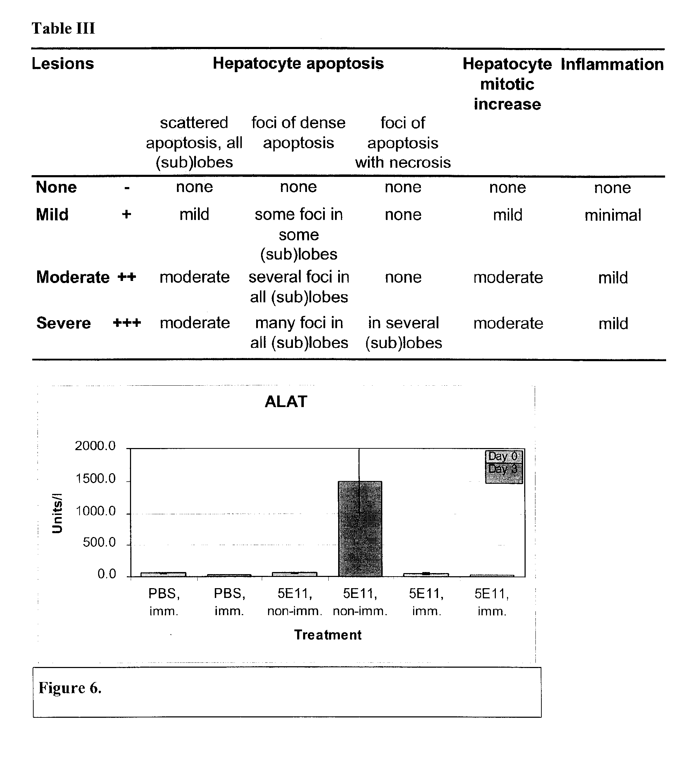

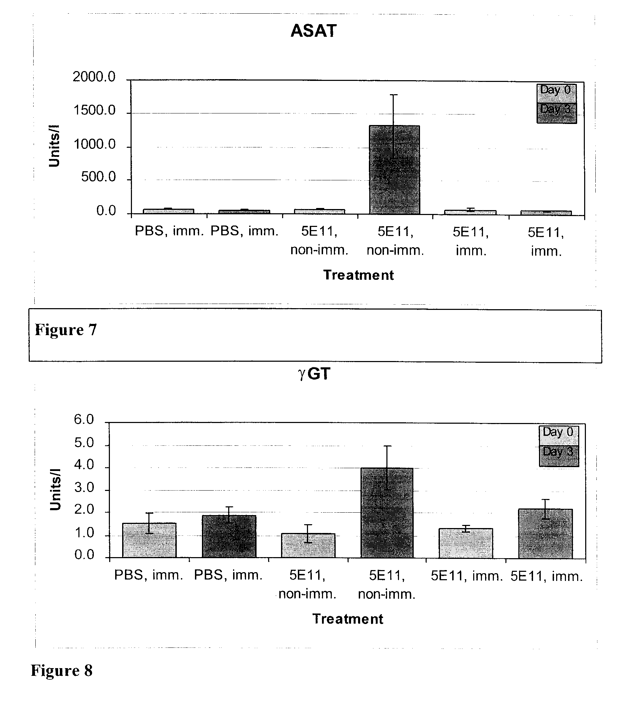

In all animals of group 2 (virus-treated, non-immunised) elevations of ALAT, ASAT, γGT and AP were detected 3 days after HAI. These elevations were significant in all cases (ANOVA-values: ALAT (p=0.0012), ASAT (p=0.0020), γGT (p=0.0182), AP levels (p=0.0001)) Graphic representations are depicted in Figures 6 to 9. Statistical evaluation was done using the computer statistical software Sysstat.

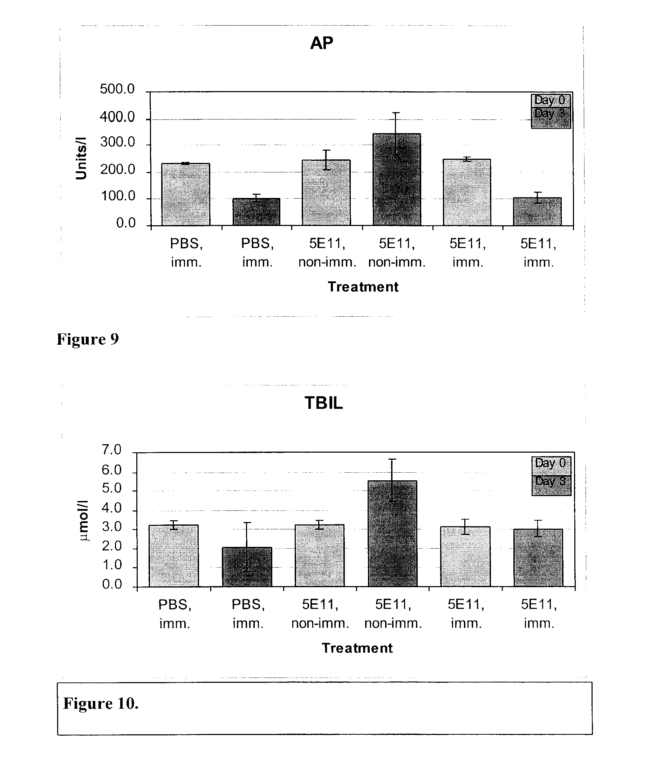

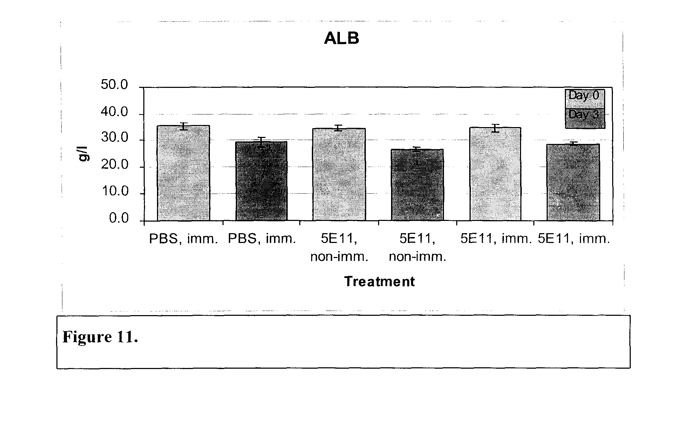

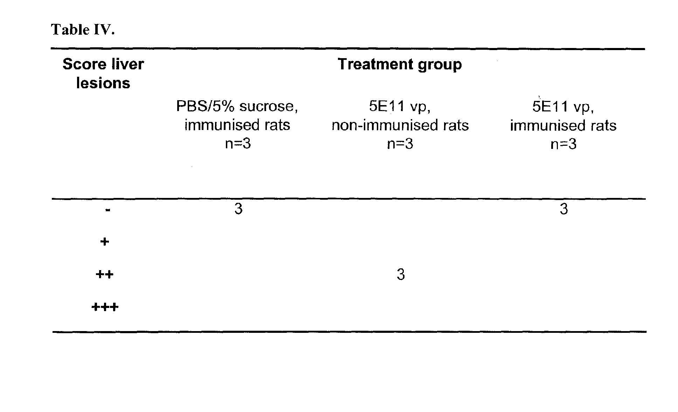

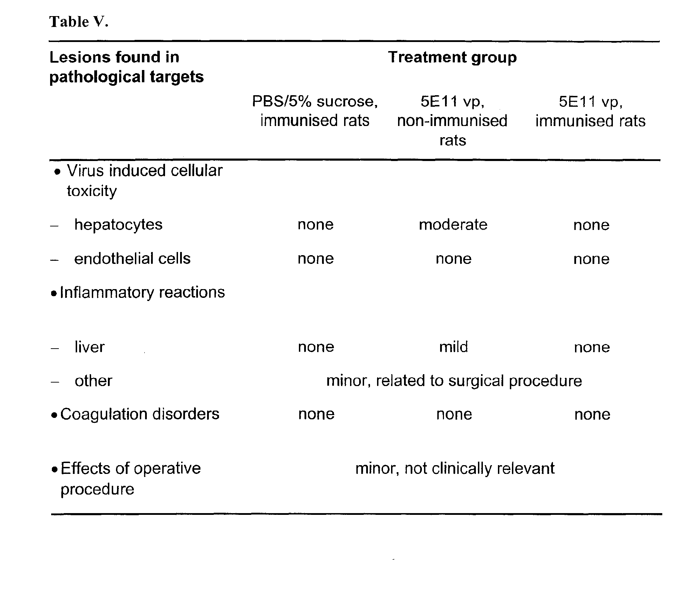

To determine the excretion and synthesising capacity of the liver after the infusion, TBIL and ALB were measured (see Figures 10 and 11). Significant elevation, and therefore, diminished excretion capacity, of TBIL was found (p=0.0007). No significant changes were seen between virus-treated naive and immunised rats for the synthesising capacity of the liver.
After necropsy, the pathological liver lesions were scored according to the earlier determined grading table. No other changes apart from the grading table were found. The incidence in liver lesions is shown in Table IV. Other lesions were minimal or not clinically relevant (see Table V).

### References

Batshaw ML, Wilson JM, Raper S, Yudkoff M, Robinson MB. Recombinant adenovirus gene transfer in adults with partial ornithine transcarbamylase deficiency (OTCD). Hum Gene Ther. 1999 Sep 20;10(14):2419-37.

Cichon G, Schmidt HH, Benhidjeb T, Loser P, Ziemer S, Haas R, Grewe N, Schnieders F, Heeren J, Manns MP, Schlag PM, Strauss M. Intravenous administration of recombinant adenoviruses causes thrombocytopenia, anemia and erythroblastosis in rabbits. J Gene Med 1999. Preprint

Connelly S, Gardner JM, Lyons RM, McClelland A, Kaleko M. Sustained expression of therapeutic levels of human factor VIII in mice. Blood. 1996 Jun 1;87(11):4671-7.

Gagandeep S, Ott M, Sokhi RP, Gupta S. Rapid clearance of syngeneic transplanted hepatocytes following transduction with E-1-deleted adenovirus indicates early host immune responses and offers novel ways for studying viral vector, target cell and host interactions. Gene Ther. 1999 May;6(5):729-36.

Lieber A, He CY, Meuse L, Schowalter D, Kirillova I, Winther B, Kay MA. The role of Kupffer cell activation and viral gene expression in early liver toxicity after infusion of recombinant adenovirus vectors. J Virol. 1997
Nov;71(11):8798-807.

Muruve DA, Barnes MJ, Stillman IE, Libermann TA. Adenoviral gene therapy leads to rapid induction of multiple chemokines and acute neutrophil-dependent hepatic injury in vivo. Hum Gene Ther. 1999 Apr 10;10(6):965-76.

Nunes FA, Furth EE, Wilson JM, Raper SE. Gene transfer into the liver of nonhuman primates with El-deleted recombinant adenoviral vectors: safety of readministration. Hum Gene Ther. 1999 Oct 10;10(15):2515-26.
Schowalter DB, Himeda CL, Winther BL, Wilson CB, Kay MA. Implication of interfering antibody formation and apoptosis as two different mechanisms leading to variable duration of adenovirus-mediated transgene expression in immune-competent mice. J Virol. 1999 Jun;73(6):4755-66.

Eastham, J.A., et al., Prostate cancer gene therapy: herpes simplex virus thymidine kinase gene transduction followed by ganciclovir in mouse and human prostate cancer models. Hum Gene Ther, 1996. 7(4): p. 515-23.

Tang, D.C., S.A. Johnston, and D.P. Carbone, Butyrate-inducible and tumor-restricted gene expression by adenovirus vectors. Cancer Gene Ther, 1994. 1(1): p. 15-20.

Esandi, M.C., et al., Gene therapy of experimental malignant mesothelioma using adenovirus vectors encoding the HSVtk gene. Gene Ther, 1997. 4(4): p. 280-7.

Vincent, A.J., et al., Treatment of leptomeningeal metastases in a rat model using a recombinant adenovirus containing the HSV-tk gene. J Neurosurg, 1996a. 85(4): p. 648-54.

Vincent, A.J., et al., Herpes simplex virus thymidine kinase gene therapy for rat malignant brain tumors. Hum Gene Ther, 1996b. 7(2): p. 197-205.

Yang, Y. and e. al, Cellular immunity to viral antigens limits E1-deleted adenoviruses for gene therapy. Proc. Natl. Acad. Sci. USA, 1994. 91: p. 4407-4411.

Mack, C.A., et al., Circumvention of anti-adenovirus neutralizing immunity by administration of an adenoviral vector of an alternate serotype. Hum Gene Ther, 1997. 8(1): p. 99-109.

Bouvet, M., et al., Suppression of the immune response to an adenovirus vector and enhancement of intratumoral transgene expression by low-dose etoposide. Gene Ther, 1998. 5(2): p. 189-95.

Kaplan, J.M. and A.E. Smith, Transient immunosuppression with deoxyspergualin improves longevity of transgene expression and ability to readminister adenoviral vector to the mouse lung. Hum Gene Ther, 1997. 8(9): p. 1095-104.

Schulick, A.H., et al., Established immunity precludes adenovirus-mediated gene transfer in rat carotid arteries. Potential for immunosuppression and vector engineering to overcome barriers of immunity. J Clin Invest, 1997. 99(2): p. 209-19.

Li, Z., et al., Efficacy of multiple administrations of a recombinant adenovirus expressing wild-type p53 in an immune-competent mouse tumor model. Gene Ther, 1998. 5(5): p. 605-13.

Molnar-Kimber, K.L., et al., Impact of preexisting and induced humoral and cellular immune responses in an adenovirus-based gene therapy phase I clinical trial for localized mesothelioma. Hum Gene Ther, 1998. 9(14): p. 2121-33.

Harvey, B.G., et al., Variability of human systemic humoral immune responses to adenovirus gene transfer vectors administered to different organs. J Virol, 1999. 73(8): p. 6729-42.

Bramson, J., et al., Pre-existing immunity to adenovirus does not prevent tumor regression following intratumoral administration of a vector expressing IL-12 but inhibits virus deissemination. Gene Ther., 1997. 4: p. 1069-1076.

Esandi, M.C., et al., IL-1/IL-3 gene therapy of non-small cell lung cancer (NSCLC) in rats using 'cracked' adenoproducer cells. Gene Ther, 1998. 5(6): p. 778-88.

Esandi, M.C., et al., Cloning, biological characterization and high-level expression of rat interleukin-3 using recombinant adenovirus: description of a new splicing variant. Gene, 1998. 211(1): p. 151-8.
Fortunati, E., et al., In vitro and in vivo gene transfer to pulmonary cells mediated by cationic liposomes. Biochim Biophys Acta, 1996. 1306(1): p. 55-62.

Although the application has been described with reference to certain preferred embodiments and illustrative examples, the scope of the invention is to be determined by reference to the appended claims.

### Table I.

Experimental design toxicity study

### Table II.

Scoring of organ lesions 3 days after virus administration via the hepatic artery.

### Figure 5.

The arrows indicate apoptotic and mitotic hepatocytes.

### Table III.

Pathological grading of liver lesions 3 days after virus administration via the hepatic artery.

### Figure 6.

ALAT levels before and 3 days after HAI in all groups

### Figure 7.

ASAT levels before and 3 days after HAI in all groups

### Figure 8.

γGT levels before and 3 days after HAI in all groups

### Figure 9.

AP levels before and 3 days after HAI in all groups

### Figure 10.

TBIL levels before and 3 days after HAI in all groups

### Figure 11.

ALB levels before and 3 days after HAI in all groups

### Table IV.

Incidence of liver lesions for the experimental groups.

### Table V.

Lesions found in pathological targets defined before the esperiment.

## Claims

1. A kit of parts for gene delivery to a recipient cell in a host, using a gene delivery vehicle to which a humoral response can be raised, comprising a nucleic acid comprising a gene to be delivered to said recipient cell and further comprising a second composition of a second gene delivery vehicle essentially identical to said first gene delivery vehicle, but preferably lacking said gene to be delivered.

2. A kit of parts according to claim 1, wherein said second composition is a vaccine and wherein said first composition is a composition for gene therapy.

3. A kit of parts according to claim 2, wherein said vaccine is to be administered at a time before said gene therapy composition at a moment in time sufficient for the host to raise a neutralising humoral response to said second gene delivery vehicle.

4. A kit of parts according to any one of claims 1-3, wherein said second gene delivery vehicle comprises a nucleic acid encoding an indifferent protein.

5. A kit of parts according to any one of claims 1-4, wherein a gene delivery vehicle is of adenoviral origin.

6. A kit of parts according to any one of claims 2-5, wherein said gene therapy composition comprises a number of first gene delivery vehicles above the number that can be neutralised by a humoral response of said host.

7. A method for delivering a gene of interest to a recipient cell in a host using a gene delivery vehicle comprising a nucleic acid comprising said gene of interest, said method comprising administering to a host a vaccine composition comprising a gene delivery vehicle lacking said gene to be delivered, allowing for a neutralising humoral response to be raised by said host to said gene delivery vehicle lacking said gene to be delivered and administering a composition for gene therapy comprising essentially the same gene delivery vehicle having a nucleic acid comprising said gene to be delivered in an amount greater than the amount which can be neutralised by said humoral response.

8. A method for avoiding or diminishing liver toxicity in a host of a gene delivery composition upon administration, comprising administering to a host a vaccine composition comprising a gene delivery vehicle, preferably lacking said gene to be delivered, allowing for a neutralising humoral response to be raised by said host to said gene delivery vehicle lacking preferably said gene to be delivered and administering a composition for gene therapy comprising essentially the same gene delivery vehicle having a nucleic acid comprising said gene to be delivered in an amount greater than the amount which can be neutralised by said humoral response.

9. A method according to claim 7 or 8, wherein said gene delivery vehicle is of adenoviral origin.

10. A kit of parts according to any one of claims 1-6 in the preparation of a pharmaceutical for the use as a pharmaceutical.

11. Use of a kit of parts according to any one of claims 1-6 in the preparation of pharmaceutical for the treatment of diseases associated with uncontrolled proliferation of cells in a host, such as tumors or autoimmune diseases.

12. Use of a kit of parts according to any one of claims 1-6 in the preparation of a pharmaceutical for the treatment of diseases associated with genetic defects in a host.

13. A kit of parts according to any one of claims 1-6, wherein said gene of interest is interleukin-3.

14. A method for the preparation of a kit of parts according to anyone of claims 1-6, comprising preparing at least one gene delivery vehicle by inserting a gene of interest in a nucleic acid to be delivered to a host cell, packaging said nucleic acid in a vehicle capable of entering a host cell and bringing a resulting gene delivery vehicle in a medium suitable for administration to a host.

15. A method according to claim 14, wherein said nucleic acid is based on or derived from an adenovirus.

16. A method according to claim 14 or 15, wherein said vehicle comprises adenoviral structural proteins.

17. A method according to claim 16, wherein at least one vehicle comprises proteins from adenoviruses of different serotypes.

18. A method according to any one of claims 15-17, wherein said nucleic acid comprises at least one ITR and a packaging signal based on or derived from an adenovirus.

19. A method according to any one of claims 15-18, wherein said nucleic acid does not encode functional structural adenoviral proteins.

20. A method according to any one of claims 15-19, wherein said nucleic acid encodes E2A adenoviral gene product.

21. A method according to claim 20, wherein said E2A gene product is temperature sensitive.

22. A method according to any one of claims 14-21, wherein said packaging occurs in a packaging cell.

23. A method according to claim 22 wherein said packaging cell is derived from or based on a primary cell.

24. A method according to claim 23, wherein said cell is based on or derived from PER.C6.

25. A method according to any one of claims 15-18 wherein said nucleic acid comprising said gene of interest is produced by a recombination step from two adenoviral vectors.

26. A method according to any one of claims 15-25, wherein said adenovirus is an adenovirus serotype 5 or serotype 35.
